(19) Europäisches Patentamt
European Patent Office
Office européen des brevets

(11) Publication number: **0 320 539 B1**

(12) **EUROPEAN PATENT SPECIFICATION**

(45) Date of publication of patent specification: **02.06.93**

(51) Int. Cl.5: **C07C 205/44**, C07C 201/16

(21) Application number: **87310950.8**

(22) Date of filing: **14.12.87**

The file contains technical information submitted after the application was filed and not included in this specification

# (54) Adsorptive separation of nitrobenzaldehyde isomers.

(43) Date of publication of application:
**21.06.89 Bulletin 89/25**

(45) Publication of the grant of the patent:
**02.06.93 Bulletin 93/22**

(84) Designated Contracting States:
**DE FR GB IT NL SE**

(56) References cited:
**US-A- 4 714 783**

**PATENT ABSTRACTS OF JAPAN, vol. 10, no. 318 (C-381)[2374], 29th October 1986, & JP-A-61 130 259**

(73) Proprietor: **UOP**
**25 East Algonquin Road**
**Des Plaines, Illinois 60017-5017(US)**

(72) Inventor: **Zinnen, Hermann A.**
**1137 Maple Avenue Apt. 2 West**
**Evanston Illinois 60202(US)**
Inventor: **Franczyk, Thad S.**
**5305 Harwood Street**
**Skokie Illinois 60077(US)**

(74) Representative: **Brock, Peter William et al**
**UROUHART-DYKES & LORD 91 Wimpole Street**
**London W1M 8AH (GB)**

EP 0 320 539 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the separation of chemical isomers and more specifically, to the separation of nitrobenzaldehyde isomers through the employment of a bed of adsorbent.

There are several ways known for separating a mixture of the isomers of nitrobenzaldehyde. However, because the decomposition temperature is so near the distillation temperature (b.p.), for safety reasons, a distillation method is ruled out. Moreover, the high boiling points make separation by distillation energy-intensive. Fractional crystallization is not satisfactory because the melting points are very close (ortho: 42-44°C; meta: 58°C). Therefore, indirect methods are used, e.g., conversion of the isomers into acetals, which can be separated by distillation, as disclosed in US-A-4450297, and then converted back into the nitrobenzaldehyde. A method of separating the isomers directly, avoiding costly chemical conversions and reconversion, is still desired.

The known methods of making nitrobenzaldehydes result in mixtures of the isomers. For example, mixtures of ortho-nitrobenzaldehyde and meta-nitrobenzaldehyde prepared by the method of Davey and Gwilt, J. Chem. Soc. 1950, 208, comprise up to 80% of ortho-nitrobenzaldehyde; up to 20% of meta-nitrobenzaldehyde and a small amount of para-nitrobenzaldehyde.

It is known from JP-A-61-130259 that nitrobenzaldehyde isomers can be separated by an adsorption-desorption process. The adsorbent is a Y-zeolite, and the desorbent is preferably a nitro-substituted aromatic compound or aldehyde-substituted aromatic compound.

We have now found that certain X- and Y-zeolites with specific alkali metal or alkaline earth metal ions at the cation-exchangeable sites will effect a separation of nitrobenzaldehyde isomers from each other by preferentially adsorbing one of the isomers, and that recovery of one of the isomers thereof is effected by desorbing the selectively adsorbed isomer with particular desorbing agents.

In its broadest aspect the present invention provides a process for separating the isomers of nitrobenzaldehyde from a feed mixture comprising at least two isomers of nitrobenzaldehyde, by contacting said mixture under adsorption conditions with an adsorbent comprising X-type zeolite having sodium or lithium cations at exchangeable cationic sites, or a Y-type zeolite having sodium, lithium, potassium, magnesium or calcium cations at the cation exchangeable sites, or a crystalline aluminium phosphate zeolite, or mixtures thereof, thereby selectively adsorbing one of said isomers to the substantial exclusion of the other isomers, and recovering the selectively adsorbed nitrobenzaldehyde isomer as an extract stream by contacting said adsorbent at desorption conditions with a desorbent material selected as follows: 1) for the X-zeolite and the aluminium phosphate zeolite, the desorbent is methyl acetate, methyl formate, benzaldehyde, ethyl acetate, acetonitrile or a mixture thereof, and 2) for the Y-zeolite, the desorbent is methyl acetate, ethyl acetate, acetonitrile, methyl formate and mixtures thereof.

In one embodiment, meta-nitrobenzaldehyde is separated from a feed mixture comprising meta-nitrobenzaldehyde and at least one other isomer of nitrobenzaldehyde. The adsorbent comprises an X-type zeolite having sodium or lithium cations at exchangeable cationic sites. The meta-isomer is selectively adsorbed to the substantial exclusion of the para and ortho-isomers, and para- and ortho-nitrobenzaldehyde are recovered in the raffinate stream.

In a second embodiment, ortho-nitrobenzaldehyde is separated from a feed mixture comprising ortho-nitrobenzaldehyde and at least one other isomer of nitrobenzaldehyde. The adsorbent comprises a Y-type zeolite having alkali metal or alkaline earth metal cations at exchangeable cationic sites or a phosphate substituted crystalline aluminium phosphate zeolite, selectively adsorbing the ortho-isomer to the substantial exclusion of the remaining isomers, removing the remaining isomers from contact with the adsorbent, and thereafter recovering high purity ortho-nitrobenzaldehyde.

Figures 1 to 12 of the accompanying Drawings are chromatographic traces of the pulse tests described in the Examples, illustrating the separations achieved with various zeolites and desorbents, and will be described in more detail in the Examples.

The following definitions of various terms used throughout this specification will be used in describing the operation, objects and advantages of the present invention.

A "feed mixture" is a mixture containing one or more extract components and one or more raffinate components to be fed to an adsorbent. The term "feed stream" indicates a stream of feed mixture which passes to an adsorbent.

An "extract component" is a type of compound or a compound, such as a particular isomer, that is more selectively adsorbed by the adsorbent while a "raffinate component" is a compound or type of compound that is less selectively adsorbed. In this process, the ortho- or meta-isomers are respective extract components and the meta- and para-isomer or ortho- and para-isomers are respective raffinate components. The term "raffinate stream" or "raffinate output stream" means a stream in which a raffinate

component is removed from an adsorbent. The composition of the raffinate stream can vary from essentially 100% desorbent material (hereinafter defined) to essentially 100% raffinate components. The term "extract stream" or "extract output stream" means a stream in which an extract material which has been desorbed by a desorbent material is removed from the adsorbent. The composition of the extract stream, likewise, can vary from essentially 100% desorbent material to essentially 100% extract components. Although it is possible by the process of this invention to produce high-purity extract product (hereinafter defined) or a raffinate product (hereinafter defined) at high recoveries, it will be appreciated that an extract component is never completely adsorbed by the adsorbent, nor is a raffinate component completely non-adsorbed by the adsorbent. Therefore, small amounts of a raffinate component can appear in the extract stream, and likewise, small amounts of an extract component can appear in the raffinate stream. The extract and raffinate streams then are further distinguished from each other and from the feed mixture by the ratio of the concentrations of an extract component and a specific raffinate component, both appearing in the particular stream. For example, in one embodiment, the ratio of concentration of the more selectively adsorbed meta-isomer to the concentration of less selectively adsorbed ortho- and para-isomers will be highest in the extract stream, next highest in the feed mixture, and lowest in the raffinate stream. Likewise, the ratio of the less selectively adsorbed ortho- and para-isomers to the more selectively adsorbed meta-isomer will be highest in the raffinate stream, next highest in the feed mixture, and the lowest in the extract stream. In the other embodiment referred to above, the ratio of concentration of the more selectively adsorbed ortho-isomer to the concentration of the less selectively adsorbed meta- and para-isomers will be highest in the extract stream, next highest in the feed stream and lowest in the raffinate stream. Likewise, the ratio of the less selectively adsorbed meta- and para-isomers to the more selectively adsorbed ortho-isomer will be highest in the raffinate stream, next highest in the feed mixture and the lowest in the extract stream. The term "desorbent material" means generally a material capable of desorbing an extract component. The term "desorbent stream" or "desorbent input stream" indicates the stream by means of which desorbent material passes into the adsorbent. When the extract stream and the raffinate stream contain desorbent materials, at least a portion of the extract stream, and preferably at least a portion of the raffinate stream from the adsorbent, will be passed to separation means, typically fractionators, where at least a portion of the desorbent material will be separated under separation conditions to produce an extract product and a raffinate product. The terms "extract product" and "raffinate product" mean products produced by the process containing, respectively, an extract component and a raffinate component in higher concentrations than those found in the respective extract stream and the raffinate stream. The term "selective pore volume" of the adsorbent is defined as the volume of the adsorbent which selectively adsorbs extract components from a feed mixture. The term "non-selective void volume" of an adsorbent is the volume of an adsorbent which does not selectively retain an extract component from a feed mixture. This volume includes the cavities of the adsorbent which contain no adsorptive sites and the interstitial void spaces between adsorbent particles. The selective pore volume and the non-selective void volume are generally expressed in volumetric quantities and are of importance in determining the proper flow rates of fluid required to be passed into the process for efficient operations to take place for a given quantity of adsorbent.

Feed mixtures which can be utilized in the process of this invention will comprise at least two nitrobenzaldehyde isomers. Isomers can be characterized by reference to Formula 1 below.

**Formula 1**

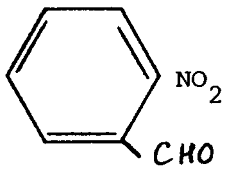

ortho-
nitrobenzaldehyde

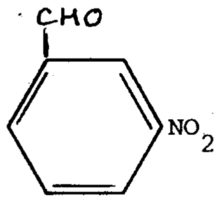

meta-
nitrobenzaldehyde

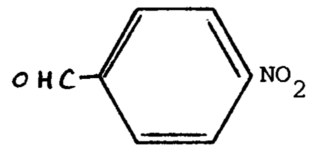

para-
nitrobenzaldehyde

The major use for ortho-nitrobenzaldehyde is in the manufacture of coronary-dilating pharmaceuticals. The major outlets for meta-nitrobenzaldehyde are in the manufacture of pharmaceuticals, dyes, surface active agents, chlorophyll, antioxidant and mosquito repellant.

To separate a first nitrobenzaldehyde isomer from a feed mixture containing that isomer and at least one other nitrobenzaldehyde isomer, the mixture is contacted with the appropriate adsorbent, whereby the first isomer is more selectively adsorbed and retained by the adsorbent, while the other isomers are relatively unadsorbed, with the first isomer being recovered as part of the extract stream, the other isomers, being relatively unadsorbed, would be recovered as part of the raffinate stream. In the present invention, either the ortho- or meta-isomer can be recovered as the extract product, depending on the choice of adsorbent. The adsorbed isomer is recovered from the adsorbent by contacting the adsorbent with a desorbent material. The term "desorbent material" as used herein means any fluid substance capable of removing a selectively adsorbed feed component from the adsorbent. Generally, in a swing-bed system in which the selectively adsorbed feed component is removed from the adsorbent by a purge stream, desorbent material selection is not too critical and desorbent materials comprising gaseous hydrocarbons such as methane, ethane, etc., or other types of gases such as nitrogen or hydrogen may be used at elevated temperatures, or reduced pressures, or both, so as effectively to purge the adsorbed feed component from the adsorbent. However, in adsorptive separation processes which employ zeolitic adsorbents and which are generally operated continuously at substantially constant pressures and temperatures to ensure liquid phase, the desorbent material relied upon must be judiciously selected to satisfy several criteria. Firstly, the desorbent material must displace the extract components from the adsorbent at reasonable mass flow rates without itself being so strongly adsorbed as unduly to prevent the extract component from displacing the desorbent material in a succeeding adsorption cycle. Expressed in terms of the selectivity (hereinafter discussed in more detail), it is preferred that the adsorbent be more selective for the extract component with respect to a raffinate component than it is for the desorbent material with respect to a raffinate component. Secondly, desorbent materials must be compatible with the particular adsorbent and the particular feed mixture. More specifically, the desorbent must not react with either the adsorbent or any component of the feed material and must not reduce or destroy the critical selectivity of the adsorbent for the extract components with respect to the raffinate component. Desorbent materials to be used in the process of this invention should additionally be substances which are easily separable from the feed mixture that is passed into the process. After desorbing the extract components of the feed, both desorbent material and the extract components are typically removed in admixture from the adsorbent. Likewise, one or more raffinate components are typically withdrawn from the adsorbent in admixture with desorbent material, and without a method of separating at least a portion of desorbent material, such as distillation, neither the purity of the extract product nor the purity of the raffinate product would be very high. It is, therefore, contemplated that any desorbent material used in this process will have an average boiling point substantially different from that of the feed mixture, to allow separation of desorbent material from feed components in the extract and raffinate streams by simple fractionation, thereby permitting reuse of desorbent material in the process. The term "substantially different" as used herein means that the difference between the average boiling points of the desorbent material and of the feed mixture is at least 5 °C. The boiling range of the desorbent material may be higher or lower than that of the feed mixture.

In the preferred isothermal, isobaric, liquid-phase operation of the process of this invention, lower alkyl acetates, e.g., methyl acetate, ethyl acetate, propyl acetate and butyl acetate, lower alkyl formates, e.g., methyl formate and ethyl formate, acetonitrile and benzaldehyde have been found to be effective desorbents. The desorbent may be dissolved in a suitable diluent, such as toluene, so that the rate of desorption may be modified as desired.

The prior art has recognized that certain characteristics of adsorbents are highly desirable, if not absolutely necessary, to the successful operation of a selective adsorption process. Among such characteristics are: adsorptive capacity for some volume of an extract component per volume of adsorbent; the selective adsorption of an extract component with respect to a raffinate component and the desorbent material; and, sufficiently fast rates of adsorption and desorption of the extract components to and from the adsorbent.

Capacity of the adsorbent for adsorbing a specific volume of one or more extract components is, of course, a necessity; without such capacity the adsorbent is useless for adsorptive separation. Furthermore, the higher the adsorbent's capacity for an extract component, the better is the adsorbent. Increased capacity of a particular adsorbent makes it possible to reduce the amount of adsorbent needed to separate the extract component contained in a particular charge rate of feed mixture. A reduction in the amount of adsorbent required for a specific adsorptive separation reduces the cost of the separation process. It is important that the good initial capacity of the adsorbent be maintained during actual use in the separation process over some economically desirable life.

The second necessary characteristic of an adsorbent is its ability to separate components of the feed; or, in other words, that the adsorbent possess adsorptive selectivity, (B), for one component as compared to another component. Relative selectivity can be expressed not only for one feed component as compared to another but can also be expressed between any feed mixture component and the desorbent material. The selectivity, (B), as used throughout this specification is defined as the ratio of the two components of the adsorbed phase over the ratio of the same two components in the unadsorbed phase at equilibrium conditions.

Relative selectivity is shown as Equation 1 below:

**Equation 1**

$$\text{Selectivity} = (B) = \frac{[\text{vol. percent C/vol. percent D}]_A}{[\text{vol. percent C/vol. percent D}]_U}$$

where C and D are two components of the feed represented in volume percent and the subscripts A and U represent the adsorbed and unadsorbed phases, respectively. The equilibrium conditions are determined when the feed passing over a bed of adsorbent does not change composition after contacting the bed of adsorbent. In other words, there is no net transfer of material occurring between the unadsorbed and adsorbed phases.

Where selectivity of two components approaches 1.0 there is no preferential adsorption of one component by the adsorbent with respect to the other; they are both adsorbed (or non-adsorbed) to about the same degree with respect to each other. As the (B) becomes less than or greater than 1.0 there is a preferential adsorption by the adsorbent for one component with respect to the other. When comparing the selectivity by the adsorbent of one component C over component D, a (B) larger than 1.0 indicates preferential adsorption of component C within the adsorbent. A (B) less than 1.0 would indicate that component D is preferentially adsorbed leaving an unadsorbed phase richer in component C and an adsorbed phase richer in component D. While separation of an extract component from a raffinate component is theoretically possible when the selectivity of the adsorbent for the extract component with respect to the raffinate component just exceeds a value of 1.0, it is preferred that such selectivity have a value approaching or exceeding 2. Like relative volatility, the higher the selectivity, the easier the separation is to perform. Higher selectivities permit a smaller amount of adsorbent to be used in the process. Ideally, desorbent materials should have a selectivity equal to 1, or less than 1, with respect to all extract components so that all of the extract components can be extracted as a class and all raffinate components clearly rejected into the raffinate stream

The third important characteristics is the rate of exchange of the extract component of the feed mixture material with the desorbent material or, in other words, the relative rate of desorption of the extract component. This characteristic relates directly to the amount of desorbent material that must be employed to recover the extract component from the adsorbent; faster rates of exchange reduce the amount of

desorbent material needed to remove the extract component and, therefore, permit a reduction in the operating cost of the process. With faster rates of exchange, less desorbent material has to be pumped through the process and separated from the extract stream for reuse.

In order to test various adsorbents and desorbent materials with a particular feed mixture to measure the adsorbent characteristics of adsorptive capacity and selectivity and exchange rate, a dynamic testing apparatus is employed. The apparatus comprises an adsorbent chamber of approximately 70 cc volume having inlet and outlet portions at opposite ends of the chamber. The chamber is contained within a temperature control means and, in addition, pressure control equipment is used to operate the chamber at a constant predetermined pressure. Chromatographic analysis equipment can be attached to the outlet line of the chamber and used to analyze "on-stream" the effluent stream leaving the adsorbent chamber.

A pulse test, performed using this apparatus and the following general procedure, is used to determine selectivities and other data for various adsorbent systems. The adsorbent is filled to equilibrium with a particular desorbent by passing the desorbent material through the adsorbent chamber. At a convenient time, a pulse of feed containing known concentrations of a non-adsorbed tracer (n-octadecane for instance) and of the particular nitrobenzaldehyde isomers, all diluted in desorbent, is injected for a duration of several minutes. Desorbent flow is resumed, and the tracer and the aromatic isomers are eluted as in a liquid-solid chromatographic operation. The effluent can be analyzed by on-stream chromatographic equipment and traces of the envelopes of corresponding component peaks developed. Alternatively, effluent samples can be collected periodically and later analyzed separately by gas chromatography.

From information derived from the chromatographic traces, adsorbent performance can be rated in terms of capacity index for an extract component, selectivity for one isomer with respect to the other, and the rate of desorption of an extract component by the desorbent. The capacity index may be determined from the distance between the centres of the peak envelope of the selectivity adsorbed isomer and the peak envelope of the tracer component, or some other known reference point. It is expressed in terms of the volume in cubic centimeters of desorbent pumped during this time interval. Selectivity, (B), for an extract component with respect to a raffinate component may be determined from the ratio of the distance between the centres of an extract component peak envelope and the tracer peak envelope (or other reference point) compared with the corresponding distance between the centres of a raffinate component peak envelope and the tracer peak envelope. The rate of exchange of an extract component with the desorbent can generally be determined from the width of the peak envelopes at half intensity. The narrower the peak width, the faster the desorption rate. The desorption rate can also be determined from the distance between the centre of the tracer peak envelope and the disappearance of an extract component which has just been desorbed. This distance is again the volume of desorbent pumped during this time interval.

The adsorbent to be used in the process of this invention comprises specific crystalline aluminosilicates. Crystalline aluminosilicates such as those used according to the present invention include crystalline aluminosilicate cage structures in which the alumina and silica tetrahedra are intimately connected in an open three-dimensional network. The tetrahedra are crosslinked by the sharing of oxygen atoms, with spaces between the tetrahedra occupied by water molecules before partial or total dehydration of this zeolite. The dehydration of the zeolite results in crystals interlaced with cells having molecular dimensions. Thus, the crystalline aluminosilicates are often referred to as "molecular sieves" when the separation which they effect is dependent essentially upon differences between the sizes of the feed molecules as, for intance, when smaller normal paraffin molecules are separated from larger isoparaffin molecules by using a particular molecular sieve. In the process of this invention, however, the term "molecular sieves", although widely used, is not strictly suitable, since the separation of specific aromatic isomers is apparently dependent on differences in electrochemical attraction of the different isomers and the adsorbent rather than solely on physical size differences in the isomer molecules.

In hydrated form, the crystalline aluminosilicates generally encompass those zeolites represented by the Formula below:

**Formula 2**

$$M_{2/n}O:Al_2O_3:wSiO_2:yH_2O$$

where "M" is a cation which balances the electrovalence of the tetrahedra and is generally referred to as an exchangeable cationic site, "n" represents the valence of the cation, "w" represents the moles of $SiO_2$, and "y" represents the moles of water. The generalized cation "M" may comprise monovalent, divalent or trivalent cations or mixtures thereof.

The prior art (e.g. US-A-3686342) has generally recognized that adsorbents comprising the type X and the type Y zeolites can be used in certain adsorptive separation processes. These zeolites are well known to the art (e.g. US-A-2882244 and -3130007).

The type X zeolite in the hydrated or partially hydrated form can be represented in terms of mole oxides as shown in Formula 3 below:

**Formula 3**

$(0.9\ 0.2)M_{2/n}O{:}Al_2O_3{:}(2.5 \pm 0.5)SiO_2{:}yH_2O$

where "M" represents at least one cation having a valence of not more than 3, "n" represents the valence of "M", and "y" is a value up to 9 depending upon the identity of "M" and the degree of hydration of the crystal. As noted from Formula 3, the $SiO_2/Al_2O_3$ mole ratio is 2.5 - 0.5. The cation "M" may be one or more of a number of cations such as those of hydrogen, alkali metals, or alkaline earth metals, or other selected cations, and is generally referred to as an exchangeable cationic site. As the type X zeolite is initially prepared, the cation "M" is usually predominately sodium and the zeolite is, therefore, referred to as a sodium-type X zeolite. Depending upon the purity of the reactants used to make the zeolite, other cations mentioned above may be present, however, as impurities.

The type Y zeolite in the hydrated or partially hydrated form can be similarly represented in terms of mole oxides as in Formula 4 below:

**Formula 4**

$(0.9 \pm 0.2)M_{2/n}O{:}Al_2O_3{:}wSiO_2{:}yH_2O$

where "M" is at least one cation having a valence not more than 3, "n" represents the valence of "M", "w" is a value from 3 to 6, and "y" is a value up to 9, depending upon the identity of "M", and the degree of hydration of the crystal. The $SiO_2/Al_2O_3$ mole ratio for type Y structured zeolites can thus be from 3 to 6. Like the type X structured zeolite, the cation "M" may be one or more of a variety of cations but, as the type Y zeolite is intially prepared, the cation "M" is also usually predominately sodium.

Other microporous crystalline materials with which the invention can be practiced are the family of aluminophosphate compositions synthesized by hydrothermal crystallization of an aluminophosphate gel containing a molecular structure-forming template, followed by calcination to yield a microporous crystalline adsorbent with uniform pores. The template can be an organic amine or a quaternary ammonium salt. These may be prepared by the procedure described in US-A-4310440. Although others may be useful in the invention, particularly preferred is $AlPO_4$-5, having a uniform pore size of 8 A, which can be prepared as set forth in Examples 1 - 26 of said patent.

The principal characteristics of the $AlPO_4$-5 adsorbent used herein is specified as follows:

Chemical Analysis (wt. % anhydrous)

$Al_2O_3$ - 41.7

$P_2O_5$ - 57.8

Molar Ratio $P_2O_5/Al_2O_3$ = 1.00

Surface Area (1 pt. BET, $N_2$) - 365 $m^2/g$

$O_2$ Capacity (-183 °C, 13,3 kPa [100 Tor]) - 14.1%

Mean Particle Size (Coulter Counter) - 4.9 µm

The present invention is based on the discovery that the type Y zeolite with alkali metal or alkaline earth metal cations at exchangeable cation sites is more selective for the ortho-isomers of nitrobenzaldehyde than for the meta- and para-isomers, and that the type X zeolite having alkali metal cations at exchangeable cationic sites, and preferably, sodium or lithium is more selective for the meta-isomer than the other isomers.

Typically, adsorbents used in separative processes contain the crystalline material dispersed in an amorphous binder material or inorganic matrix, having channels and cavities therein which enable liquid access to the crystalline material. Silica, alumina, or mixtures thereof are typical of such inorganic matrix materials. The binder aids in forming or agglomerating the crystalline particles which otherwise would comprise a fine powder. The adsorbent may thus be in the form of particles such as extrudates, aggregates, tablets, macrospheres or granules having a desired particle range, preferably from 16 to 60 mesh (Standard U.S. Mesh) which corresponds to 0.25 to 1.19mm nominal aperture. Lower water content in the adsorbent is advantageous from the standpoint of having less water contamination of the product.

The adsorbent may be employed in the form of a dense fixed bed which is alternately contacted with a feed mixture and a desorbent material, in which case the process will be only semicontinuous. In another embodiment, a set of two or more static beds of adsorbent may be employed with appropriate valving, so that a feed mixture can be passed through one or more adsorbent beds of a set, while a desorbent material can be passed through one or more of the other beds in a set. The flow of a feed mixture and a desorbent material may be either upwards or downwards through an adsorbent in such beds. Any of the conventional apparatus employed in a static bed fluid-solid contacting may be used.

Moving bed or simulated moving bed flow system, however, have a much greater separation efficiency than fixed bed systems and are, therefore, preferred. In the moving bed or simulated moving bed processes, the retention and displacement operations are continuously taking place which allows both continuous production of an extract and a raffinate stream, and the continual use of feed and displacement fluid streams. One preferred embodiment of this process utilizes what is known in the art as the simulated moving bed countercurrent flow system. In such a system, it is the progressive movement of multiple liquid access points down a molecular sieve chamber that simulates the upward movement of molecular sieve contained in the chamber. For further explanation of the simulated moving bed countercurrent process flowscheme reference can also be made to US-A-2985589, in which the operating principles and sequence of such a flow system are described, and to a paper entitled, "Continuous Adsorptive Processing - A New Separation Technique" by D. B. Broughton presented at the 34th Annual Meeting of the Society of Chemical Engineers at Tokyo, Japan on April 2, 1969.

Another embodiment of a simulated moving bed flow system suitable for use in the process of the present invention is the cocurrent high efficiency simulated moving bed process disclosed in US-A-4402832.

It is contemplated that at least a portion of the extract output stream will pass into a separation means wherein at least a portion of the desorbent material can be separated, under separating conditions to produce an extract product containing a reduced concentration of desorbent material. Preferably, but not necessary in the operation of the process, at least a portion of the raffinate output stream will also be passed to a separation means wherein at least a portion of the desorbent material can be separated under separating conditions to produce a desorbent stream which can be reused in the process and a raffinate product containing a reduced concentration of desorbent material. Typically, the concentration of desorbent material in the extract product and the raffinate product will be less than 5 vol. %, and more preferably less than 1 vol. %. The separation means will typically be a fractionation column, the design and operation of which is well known to the separation art.

Although both liquid and vapor phase operations can be used in many adsorptive separation processes, liquid-phase operation is preferred for this process, because of the lower temperature requirements and because of the higher yields of an extract product that can be obtained with liquid-phase operation over those obtained with vapour-phase operation. Adsorption conditions will generally include a temperature of from 20 to 250 °C, with 100 to 200 °C being more preferred, and a pressure sufficient to maintain liquid phase. Desorption conditions will include the same range of temperatures and pressure as used for adsorption conditions.

The size of the units which can utilize the process of this invention can vary anywhere from those of pilot-plant scale (see for example US-A-3706812) to those of commercial scale and can range in flow rates from as little as a few cc an hour up to many thousands of litres per hour.

The following examples are presented for illustration purposes and more specifically are presented to illustrate the selectivity relationships that make the process of the invention possible.

**EXAMPLE 1**

In this experiment, the pulse test was performed to evaluate the ability of the present invention to separate isomers of nitrobenzaldehyde. The adsorbent used was a sodium-exchanged X zeolite and was mixed with 15 wt. % clay. Water was added and the resulting mixture was extruded, calcined at 400 °C, then ground to 20 to 60 mesh size (corresponding to a nominal aperture of 0.25 to 0.84 mm). The adsorbent was re-dried at 350 °C before it was utilized in the process in each test. The desorbent used was methyl acetate.

The testing apparatus was the above-described pulse test apparatus. For each pulse test, the column was maintained at a temperature of 110 °C and a pressure of 125 psig (862 kPa gauge) to maintain liquid-phase operations. Gas chromatographic analysis equipment was used to analyze the column effluent stream in order to determine the composition of the effluent material at given time intervals. The feed mixture employed for each test comprised 2 cc pulses of a mixture containing 0.5 g each of the three isomers, o-,

m- and p-nitrobenzaldehyde, 0.25 g of n-octadecane, which was used as a tracer, and 2.5 ml of the desorbent material (methyl acetate). The operations taking place for each test were as follows. The desorbent material was run continuously at a nominal liquid hourly space velocity (LHSV) of 1.0 $hr^{-1}$. At some convenient time interval, a pulse of the feed mixture was introduced. The desorbent stream was then resumed at 1 LHSV and continued to pass into the adsorbent column until all of the feed components had been eluted from the column, as determined by chromatographic analysis of the effluent material leaving the adsorption column.

The results of the test of this Example are shown on the accompanying Figure 1 which comprises the chromatographic trace. Retention volumes and selectivities derived from the traces are as follows:

| Selectivity | Retention Volumes ml |
|---|---|
| $(B)_{m/o}$ = 1.65 | META: 70.6 |
| $B_{m/p}$ = 2.76 | ORTHO: 158.2 |
| $B_{o/p}$ = 1.68 | PARA: 50.5 |

It is clear from the test that the separation of meta-nitrobenzaldehyde from the other isomers is readily achieved by the process of the present invention. There is minimal overlap between the meta-curve and curves for the ortho- and para-isomers. Selectivities for the meta-isomer relative to the ortho- and para-isomers are sufficiently high for good separation.

**EXAMPLE 2**

The test of Example 1 was repeated for several additional X-type molecular sieves. In a first test a type X sodium-exchanged zeolite and in a second test, a lithium-exchanged type X zeolite was used.

The results of the above two tests are shown on the accompanying Figures 2 and 3. The selectivities are as follows:

**Table**

| Figure | Adsorbent | Desorbent | Selectivity |
|---|---|---|---|
| 2 | NaX | Benzaldehyde | $B_{m/o}$ = 2.17 |
| | | | $B_{o/p}$ = 1.12 |
| | | | $B_{m/p}$ = 2.42 |
| 3 | LiX | Methyl acetate | $B_{m/o}$ = 1.88 |
| | | | $B_{m/p}$ = 1.58 |
| | | | $B_{p/o}$ = 1.19 |

It is clear from Figures 2 and 3 and the data in the above table that the adsorbent of the present invention exhibits acceptable selectivity for meta-nitrobenzaldehyde.

**EXAMPLE 3**

The pulse test was repeated for another group of zeolites each of which shows a preferential adsorption of the ortho-isomer. Except as noted in the following Table, the conditions were the same as in Example 1.

| Figure No. | Adsorbent | Desorbent | Selectivities $B_{o/m}$ | $B_{o/p}$ | $B_{m/p}$ | Other Comment |
|---|---|---|---|---|---|---|
| 4 | LiY | Methyl Acetate | 2.25 | 3.88 | 1.73 | |
| 5 | KY | Methyl Acetate | 1.90 | 3.23 | 1.70 | |
| 6 | NaY | Methyl Acetate | 2.17 | 3.99 | 1.84 | $SiO_2/Al_2O_3$ = 5 |
| 7 | NaY | Methyl Acetate | 1.80 | 4.60 | 2.55 | $SiO_2/Al_2O_3$ = 3.07 |
| 8 | NaY | Ethyl Acetate | 2.01 | 3.83 | 1.90 | |
| 9 | MgY | Ethyl Acetate | 2.47 | 2.47 | 1.00 | |
| 10 | CaY | Ethyl Acetate | 1.96 | 2.15 | 1.10 | temp = 104°C |
| 11 | NaY | 30% (vol.) acetonitrile 70% (vol.) Toluene | 1.75 | 1.62 | 1.08 | |
| 12 | NaY | Methyl Formate | 1.97 | 1.97 | 1.0 | temp = 80°C |

**EXAMPLE 4**

$AlPO_4$-5, which can be prepared according to the procedure set forth in US-A-4310440, Examples 1 - 26, having 8 Å pores, was mixed with a clay binder to produce an adsorbent of 20-60 mesh particle size (corresponding to a nominal aperture of 0.25 to 0.84 mm). The pulse test column was filled with the adsorbent and a 2 cc pulse of feed material containing 0.50 g each of o-nitrobenzaldehyde, m-nitrobenzaldehyde and p-benzaldehyde, 0.25 g n-octadecane and 2.5 ml desorbent was injected into the column as previously explained. Following the feed, the flow of ethyl acetate desorbent was continued. Ortho-nitrobenzaldehyde was preferentially adsorbed and was well-separated from the meta- and para-isomers as shown in Figure 13. The selectivities (B) were as follows:

$B_{o/m}$ = 6.75
$B_{o/p}$ = 4.23
$B_{p/m}$ = 1.60

## Claims

1. A process for separating the isomers of nitrobenzaldehyde from a feed mixture comprising at least two isomers of nitrobenzaldehyde, by contacting said mixture under adsorption conditions with a zeolite adsorbent to selectively adsorb one of said isomers and recovering the selectively adsorbed nitrobenzaldehyde isomer as an extract stream by contacting said adsorbent at desorption conditions with a desorbent material, characterised in that the adsorbent comprises an X-type zeolite having sodium or lithium cations at exchangeable cationic sites, or a Y-type zeolite having sodium, lithium, potassium, magnesium or calcium cations at the cation exchangeable sites, or a crystalline aluminium phosphate zeolite, or mixtures thereof, and in that the desorbent is selected as follows: 1) for the X-zeolite and the aluminium phosphate zeolite, the desorbent is methyl acetate, methyl formate, benzaldehyde, ethyl acetate, acetonitrile or a mixture thereof, and 2) for the Y-zeolite, the desorbent is methyl acetate, ethyl acetate, acetonitrile, methyl formate and mixtures thereof.

2. A process according to Claim 1 characterised in that said adsorbent is a Y-type zeolite, or an aluminium-phosphate zeolite, and said adsorbed isomer is ortho-nitrobenzaldehyde.

3. A process according to Claim 1 characterised in that said adsorbent is an X-type zeolite and said adsorbed isomer is meta-nitrobenzaldehyde.

4. A process according to any one of Claims 1 to 3 characterised in that said adsorption and desorption conditions comprise a temperature form 20 to 250 °C.

5. A process according to any one of Claims 1 to 4 characterised in that said process is effected with a simulated moving bed flow system.

## Patentansprüche

1. Verfahren zur Abtrennung der Nitrobenzaldehyd-Isomeren von einem Beschickungsgemisch, das wenigstens zwei Nitrobenzaldehyd-Isomere umfaßt, durch Behandlung dieses Gemisches unter Adsorptionsbedingungen mit einem Zeolithadsorbens, um selektiv eines der Isomeren zu adsorbieren, und Gewinnung des selektiv adsorbierten Nitrobenzaldehyd-Isomeren als einen Extraktstrom durch Behandlung des Adsorbens bei Desorptionsbedingungen mit einem Desorbensmaterial, **dadurch gekennzeichnet,** daß das Adsorbens einen Zeolith von Typ X mit Natrium- oder Lithiumkationen an austauschbaren kationischen Stellen oder einen Zeolith vom Typ Y mit Natrium-, Lithium-, Kalium-, Magnesium- oder Calciumkationen an den für Kationen austauschbaren Stellen oder einen kristallinen Aluminiumphosphatzeolith oder Gemische hiervon umfaßt und daß das Desorbens folgendermaßen ausgewählt ist:
   1. für den Zeolith X und den Aluminiumphosphatzeolith ist das Desorbens Methylacetat, Methylformiat, Benzaldehyd, Ethylacetat, Acetonitril oder ein Gemisch hiervon, und
   2. für den Zeolith Y ist das Desorbens Methylacetat, Ethylacetat, Acetontril, Methylformiat oder ein Gemisch hiervon.

2. Verfahren nach Anspruch 1, **dadurch gekennzeichnet,** daß das Adsorbens ein Zeolith vom Typ Y oder ein Aluminiumphosphatzeolith ist und das adsorbierte Isomere ortho-Nitrobenzaldehyd ist.

3. Verfahren nach Anspruch 1, **dadurch gekennzeichnet,** daß das Adsorbens ein Zeolith vom Typ X ist und das adsorbierte Isomere meta-Nitrobenzaldehyd ist.

4. Verfahren nach einem der Ansprüche 1 bis 3, **dadurch gekennzeichnet,** daß die Adsorptions- und Desorptionsbedingungen eine Temperatur von 20 bis 250 °C umfassen.

5. Verfahren nach einem der Ansprüche 1 bis 4, **dadurch gekennzeichnet,** daß das Verfahren mit einem simulierten Bewegtbettströmungssystem durchgeführt wird.

## Revendications

1. Procédé pour séparer les isomères de nitrobenzaldéhyde d'un mélange de charge comprenant au moins deux isomères de nitrobenzaldéhyde, qui consiste à mettre en contact ledit mélange, dans des conditions d'adsorption, avec un adsorbant zéolithique, pour adsorber sélectivement l'un desdits isomères et récupérer l'isomère de nitrobenzaldéhyde adsorbé sélectivement sous forme de courant d'extrait, en mettant en contact ledit adsorbant, dans des conditions de désorption, avec une matière désorbante, caractérisé en ce que l'adsorbant comprend une zéolithe de type X renfermant des cations sodium ou lithium aux sites cationiques échangeables, ou une zéolithe de type Y renfermant des cations sodium, lithium, potassium, magnésium ou calcium aux sites cationiques échangeables, ou une zéolithe de type phosphate d'aluminium cristallin, ou des mélanges de celles-ci, et en ce que le désorbant est choisi de la manière suivante : 1) pour la zéolithe X et la zéolithe de type phosphate d'aluminium, le désorbant est l'acétate d'éthyle, le formiate de méthyle, le benzaldéhyde, l'acétate d'éthyle, l'acétonitrile ou un mélange de ceux-ci, et 2) pour la zéolithe Y, le désorbant est l'acétate de méthyle, l'acétate d'éthyle, l'acétonitrile, le formiate de méthyle, et des mélanges de ceux-ci.

2. Procédé selon la revendication 1, caractérisé en ce que ledit adsorbant est une zéolithe de type Y ou une zéolithe de type phosphate d'aluminium, et ledit isomère adsorbé est l'ortho-nitrobenzaldéhyde.

3. Procédé selon la revendication 1, caractérisé en ce que ledit adsorbant est une zéolithe de type X et ledit isomère adsorbé est le méta-nitrobenzaldéhyde.

4. Procédé selon l'une quelconque des revendications 1 à 3, caractérisé en ce que lesdites conditions d'adsorption et de désorption comprennent une température de 20 à 250 °C.

5. Procédé selon l'une quelconque des revendications 1 à 4, caractérisé en ce que ledit procédé est réalisé avec un système d'écoulement à lit mobile simulé.

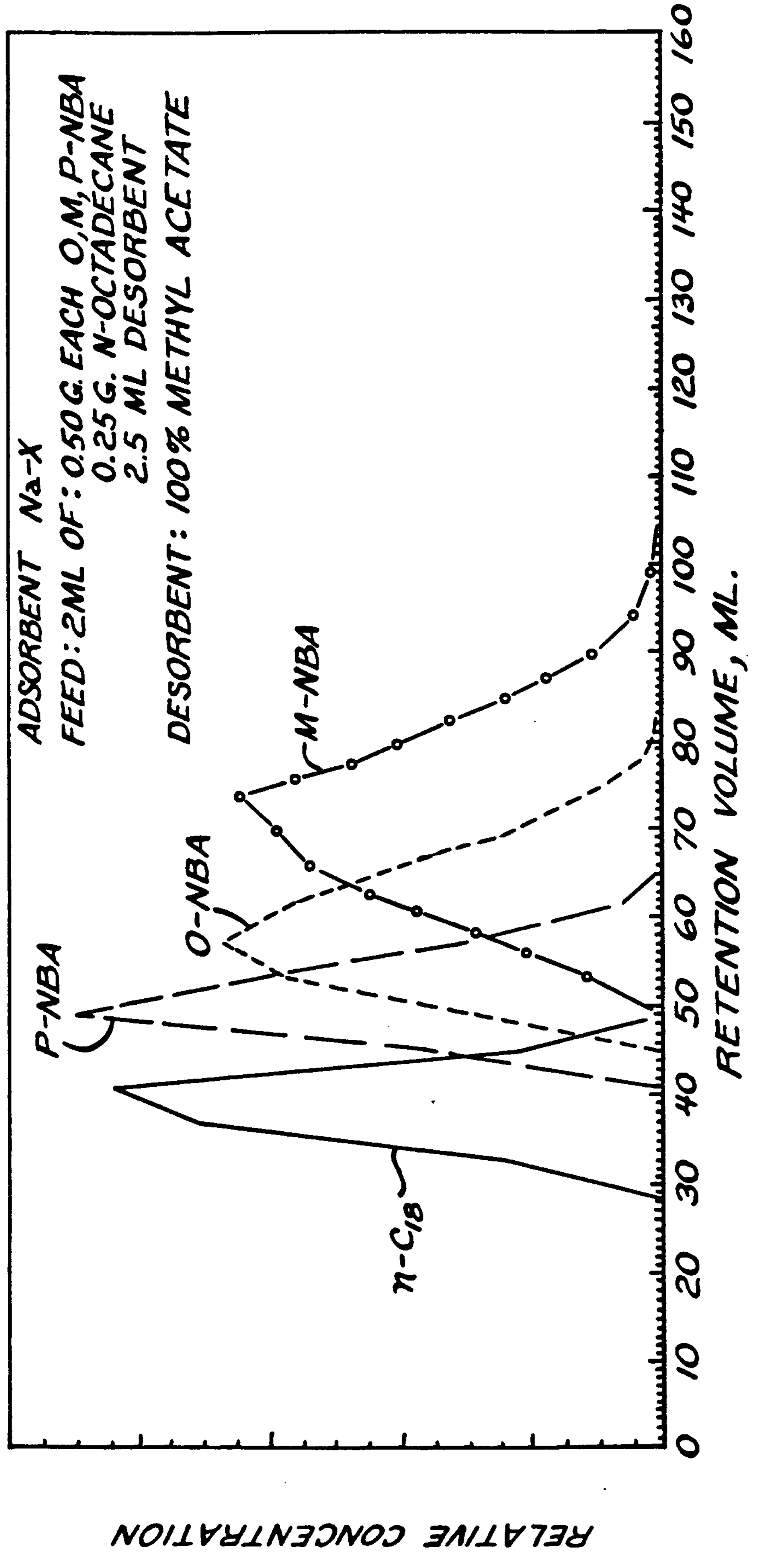
ADSORBENT Na-X
FEED: 2ML OF: 0.50 G. EACH O,M,P-NBA
0.25 G. N-OCTADECANE
2.5 ML DESORBENT
DESORBENT: 100% METHYL ACETATE
P-NBA
O-NBA
M-NBA
n-C18
RELATIVE CONCENTRATION
RETENTION VOLUME, ML.
0
10
20
30
40
50
60
70
80
90
100
110
120
130
140
150
160

FIG. 1

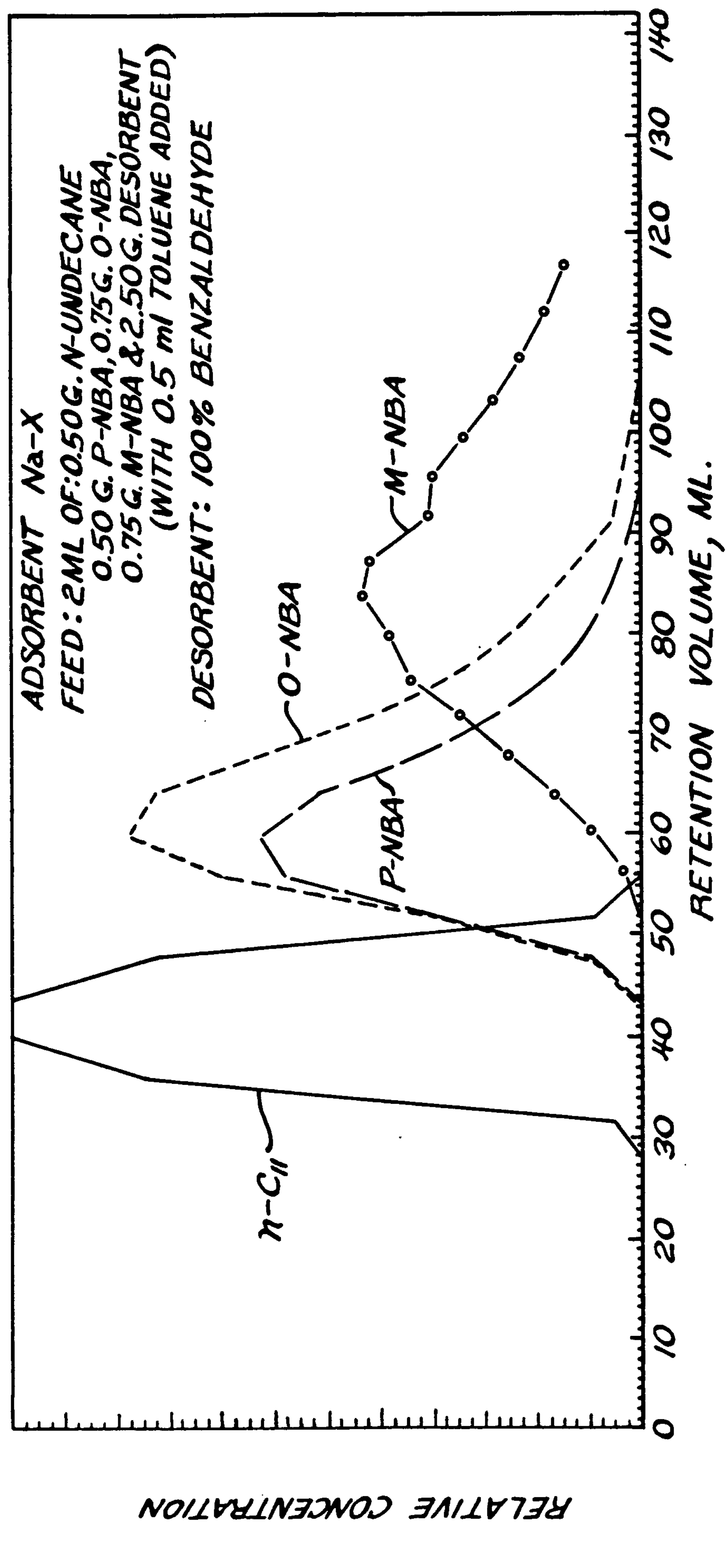
ADSORBENT Na-X
FEED: 2ML OF: 0.50G. N-UNDECANE
0.50 G. P-NBA, 0.75G. O-NBA,
0.75G. M-NBA & 2.50G. DESORBENT
(WITH 0.5 ml TOLUENE ADDED)
DESORBENT: 100% BENZALDEHYDE
O-NBA
M-NBA
P-NBA
n-C11
0
10
20
30
40
50
60
70
80
90
100
110
120
130
140
RETENTION VOLUME, ML.
RELATIVE CONCENTRATION

FIG. 2

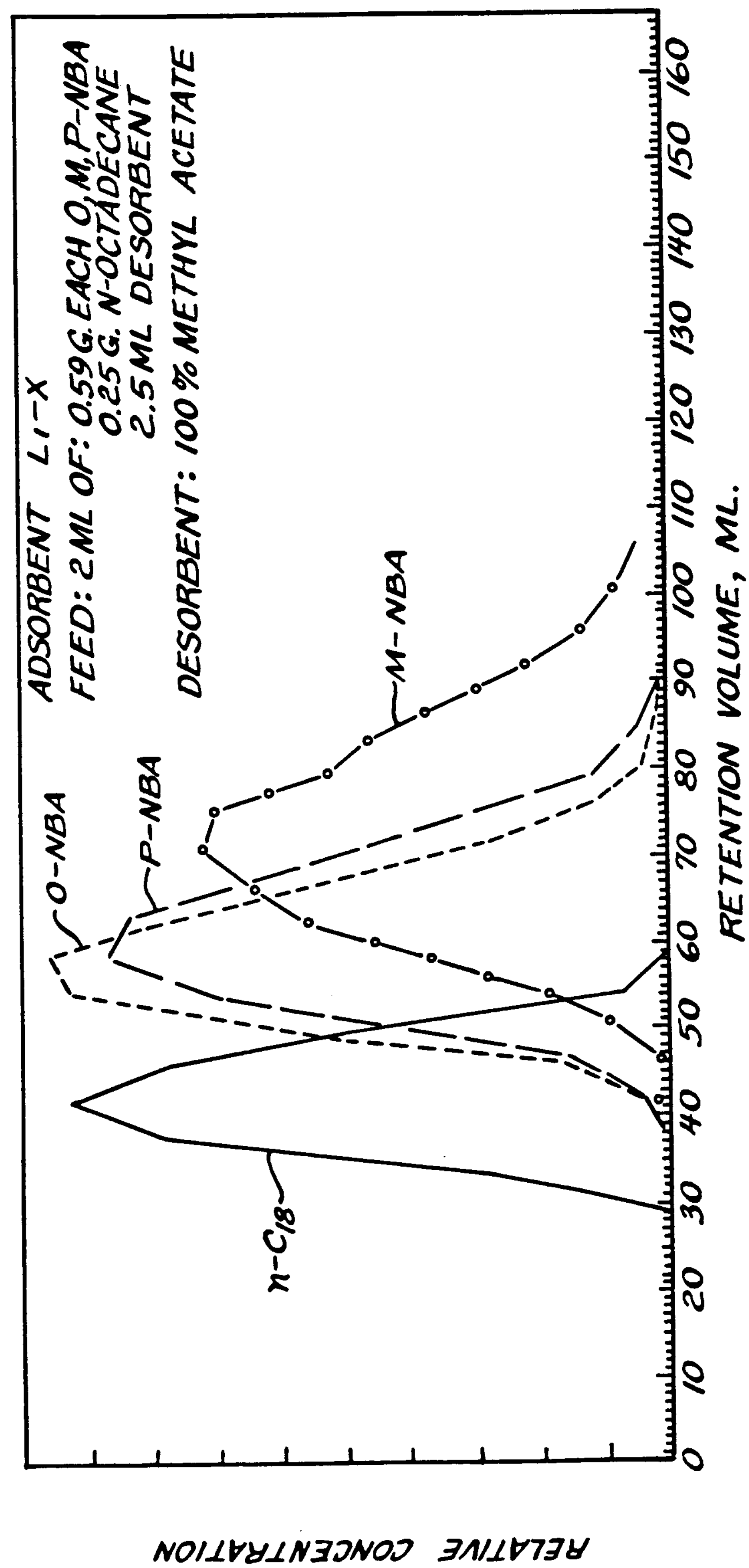
ADSORBENT Li-X
FEED: 2 ML OF: 0.59 G. EACH O,M,P-NBA
0.25 G. N-OCTADECANE
2.5 ML DESORBENT
DESORBENT: 100% METHYL ACETATE
O-NBA
P-NBA
M-NBA
n-C18
0
10
20
30
40
50
60
70
80
90
100
110
120
130
140
150
160
RETENTION VOLUME, ML.
RELATIVE CONCENTRATION

FIG. 3

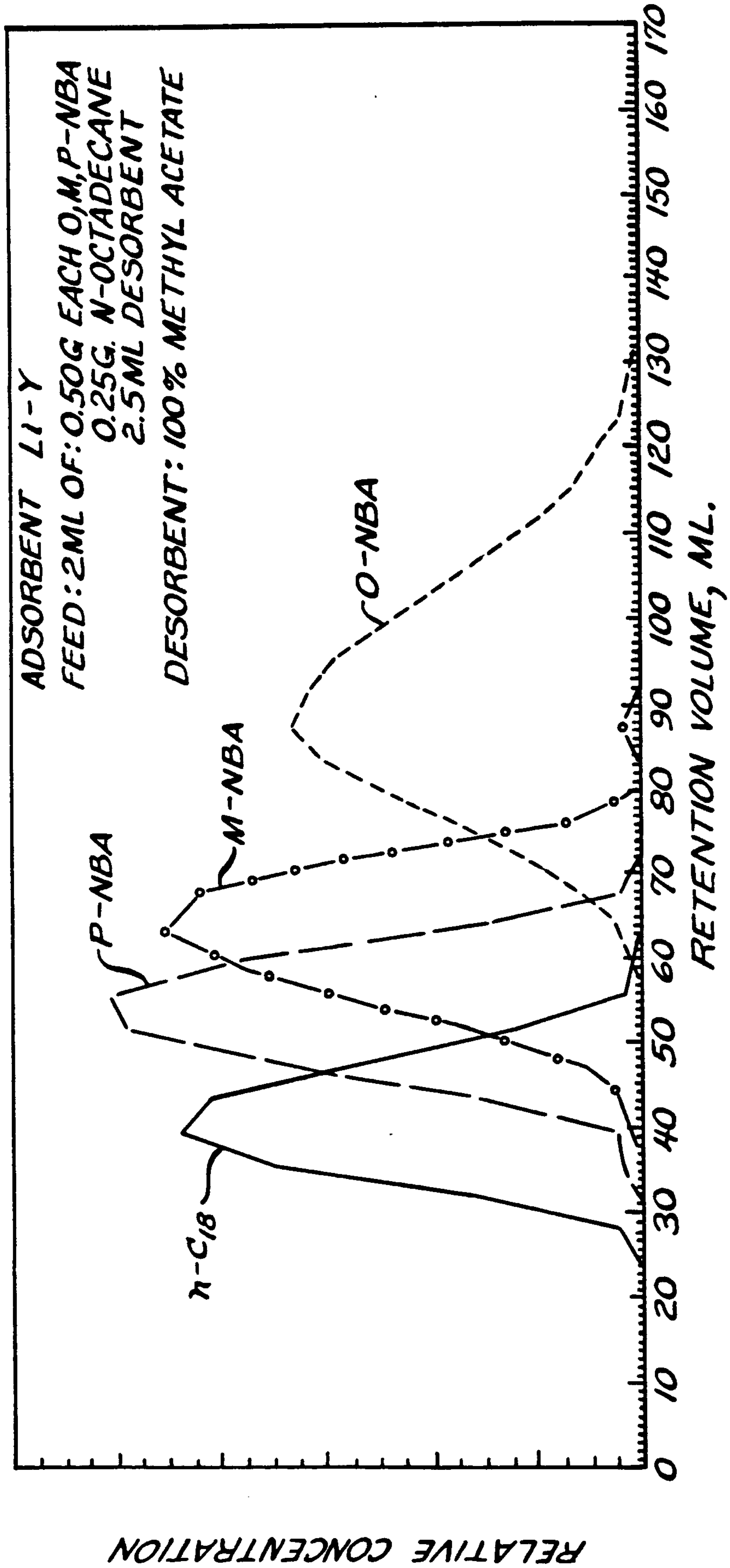
ADSORBENT LI-Y
FEED: 2ML OF: 0.50G EACH O,M,P-NBA
0.25G. N-OCTADECANE
2.5ML DESORBENT
DESORBENT: 100% METHYL ACETATE
O-NBA
M-NBA
P-NBA
$n$-$C_{18}$
0
10
20
30
40
50
60
70
80
90
100
110
120
130
140
150
160
170
RETENTION VOLUME, ML.
RELATIVE CONCENTRATION

FIG. 4

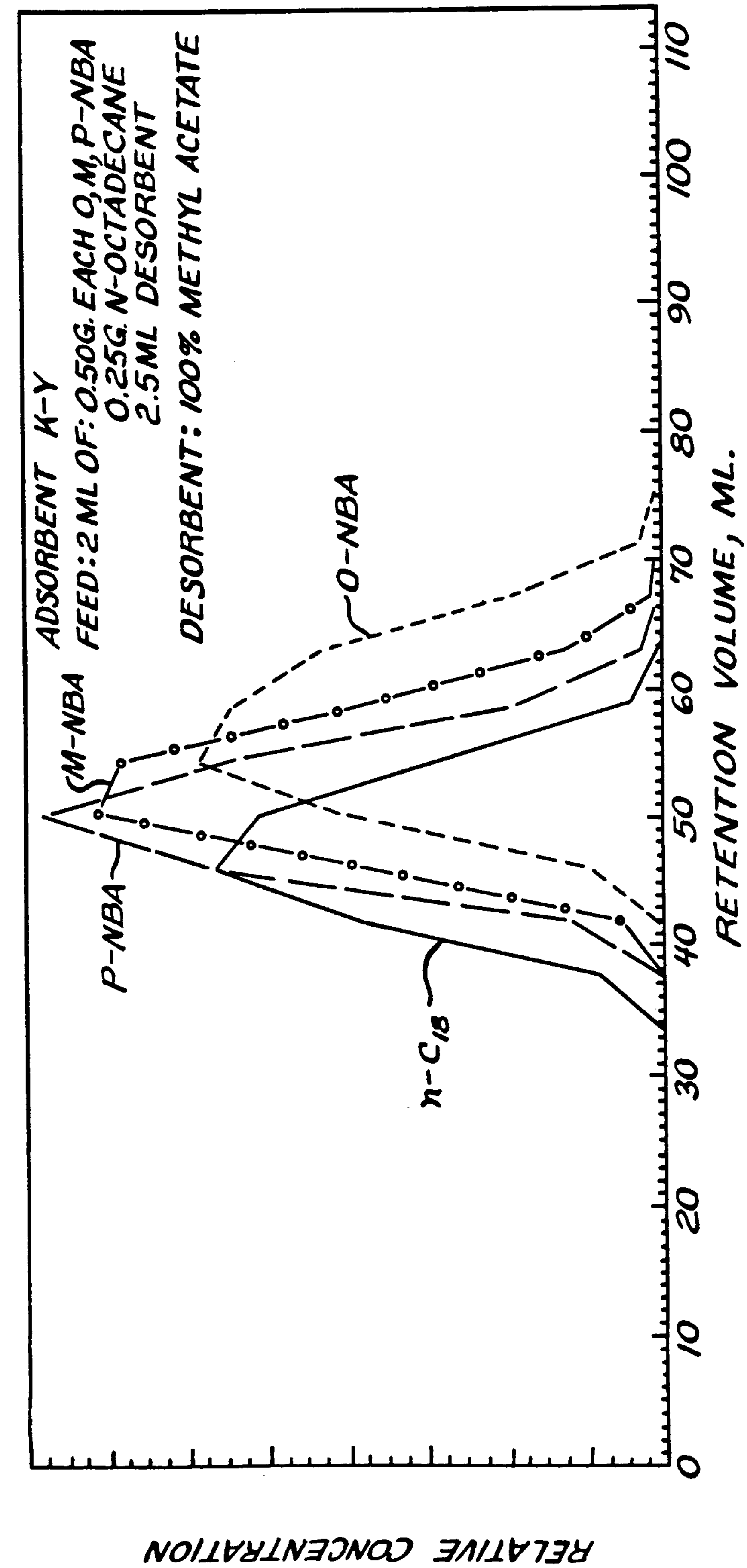
ADSORBENT K-Y
FEED: 2 ML OF: 0.50G. EACH O, M, P-NBA
0.25G. N-OCTADECANE
2.5 ML DESORBENT
DESORBENT: 100% METHYL ACETATE
M-NBA
O-NBA
P-NBA
n-C18
0
10
20
30
40
50
60
70
80
90
100
110
RETENTION VOLUME, ML.
RELATIVE CONCENTRATION

FIG. 5

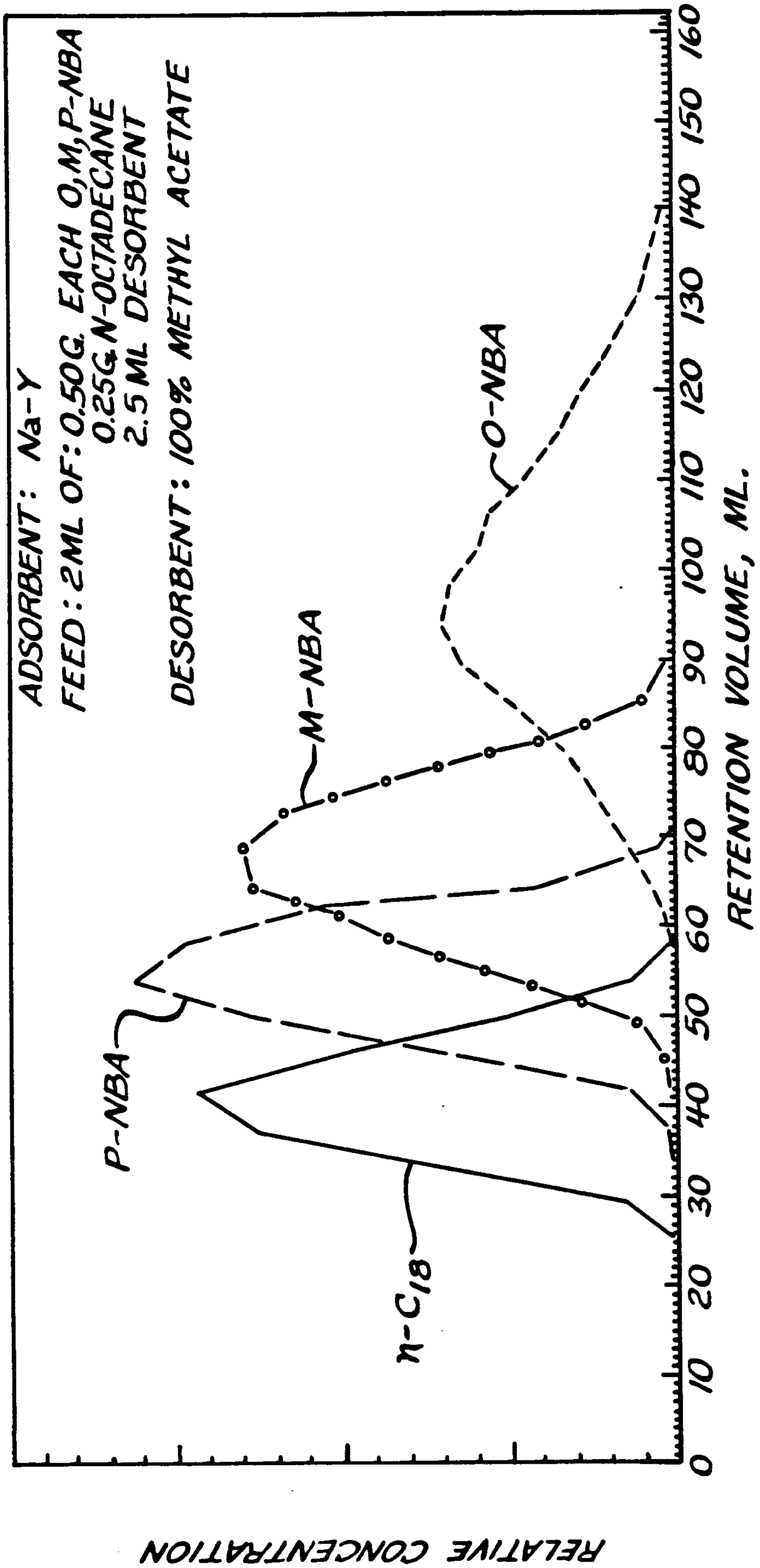
ADSORBENT: Na-Y
FEED: 2ML OF: 0.50G. EACH O,M,P-NBA
0.25G. N-OCTADECANE
2.5 ML DESORBENT
DESORBENT: 100% METHYL ACETATE
O-NBA
M-NBA
P-NBA
n-C18
RETENTION VOLUME, ML.
RELATIVE CONCENTRATION
0
10
20
30
40
50
60
70
80
90
100
110
120
130
140
150
160

FIG. 6

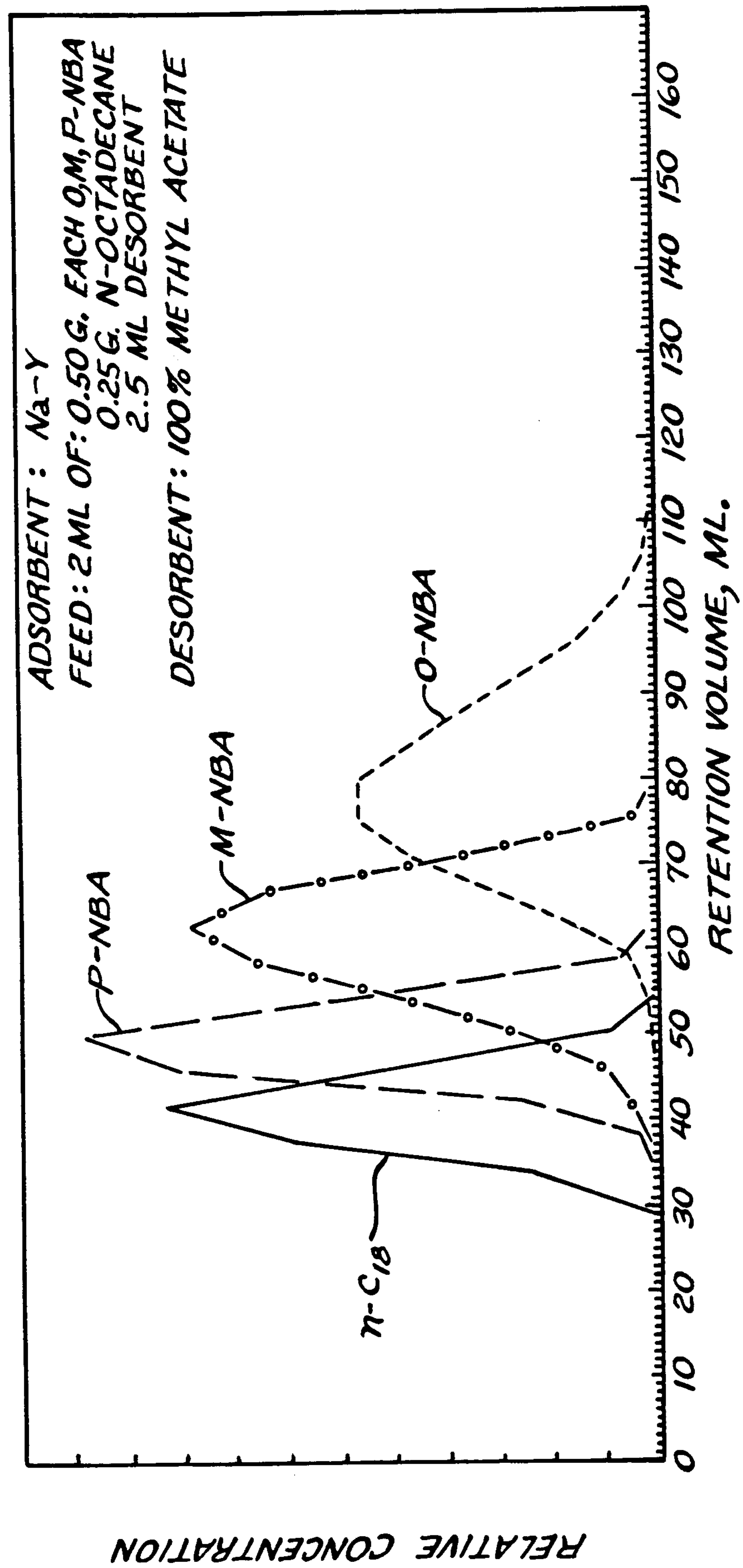
ADSORBENT : Na-Y
FEED : 2 ML OF : 0.50 G. EACH O,M,P-NBA
0.25 G. N-OCTADECANE
2.5 ML DESORBENT
DESORBENT : 100% METHYL ACETATE
P-NBA
M-NBA
O-NBA
n-C18
0 10 20 30 40 50 60 70 80 90 100 110 120 130 140 150 160
RETENTION VOLUME, ML.
RELATIVE CONCENTRATION

FIG. 7

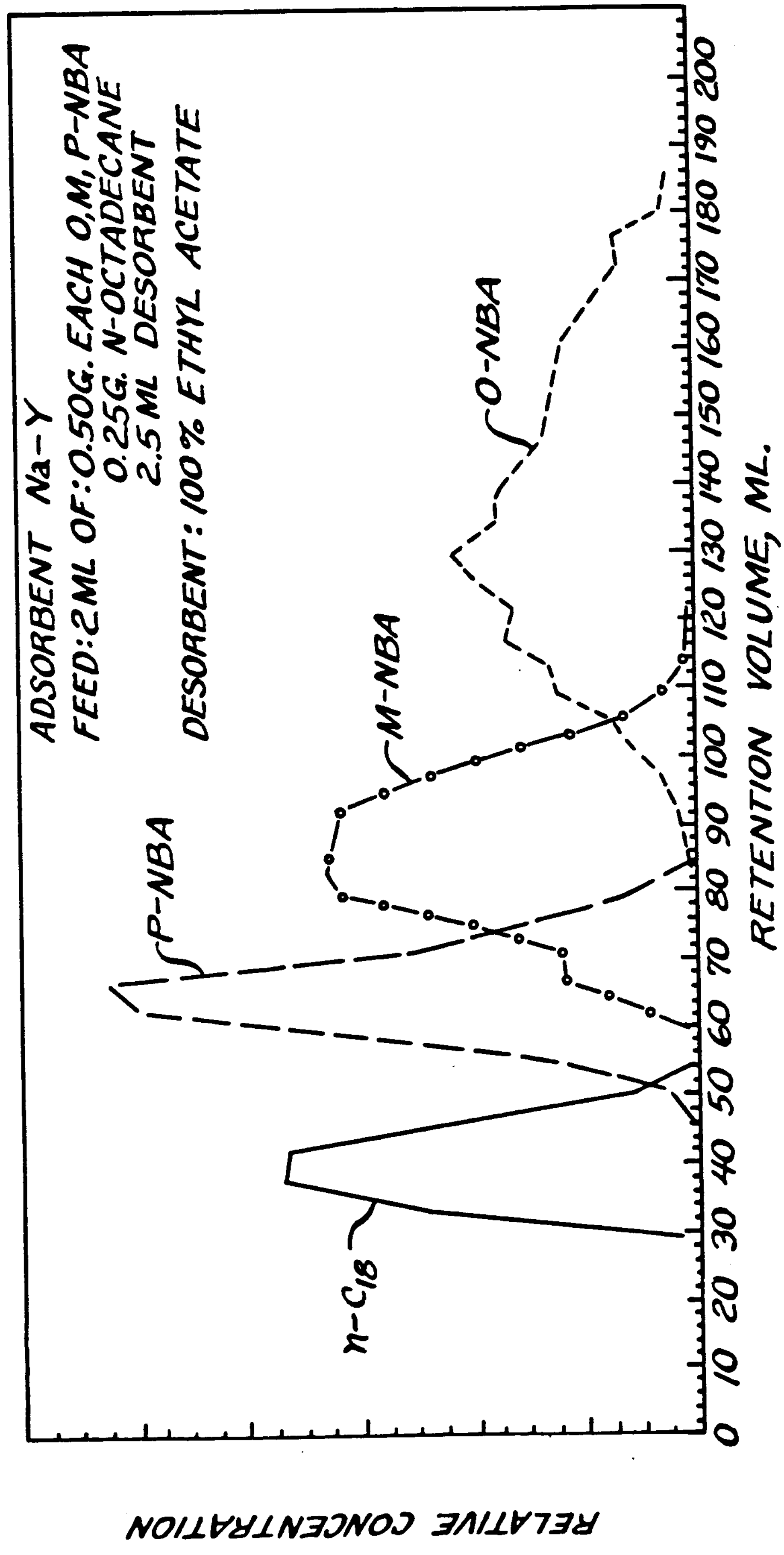
ADSORBENT Na-Y
FEED: 2ML OF: 0.50G. EACH O,M,P-NBA
0.25G. N-OCTADECANE
2.5ML DESORBENT
DESORBENT: 100% ETHYL ACETATE
P-NBA
M-NBA
O-NBA
n-C18
0 10 20 30 40 50 60 70 80 90 100 110 120 130 140 150 160 170 180 190 200
RETENTION VOLUME, ML.
RELATIVE CONCENTRATION

FIG. 8

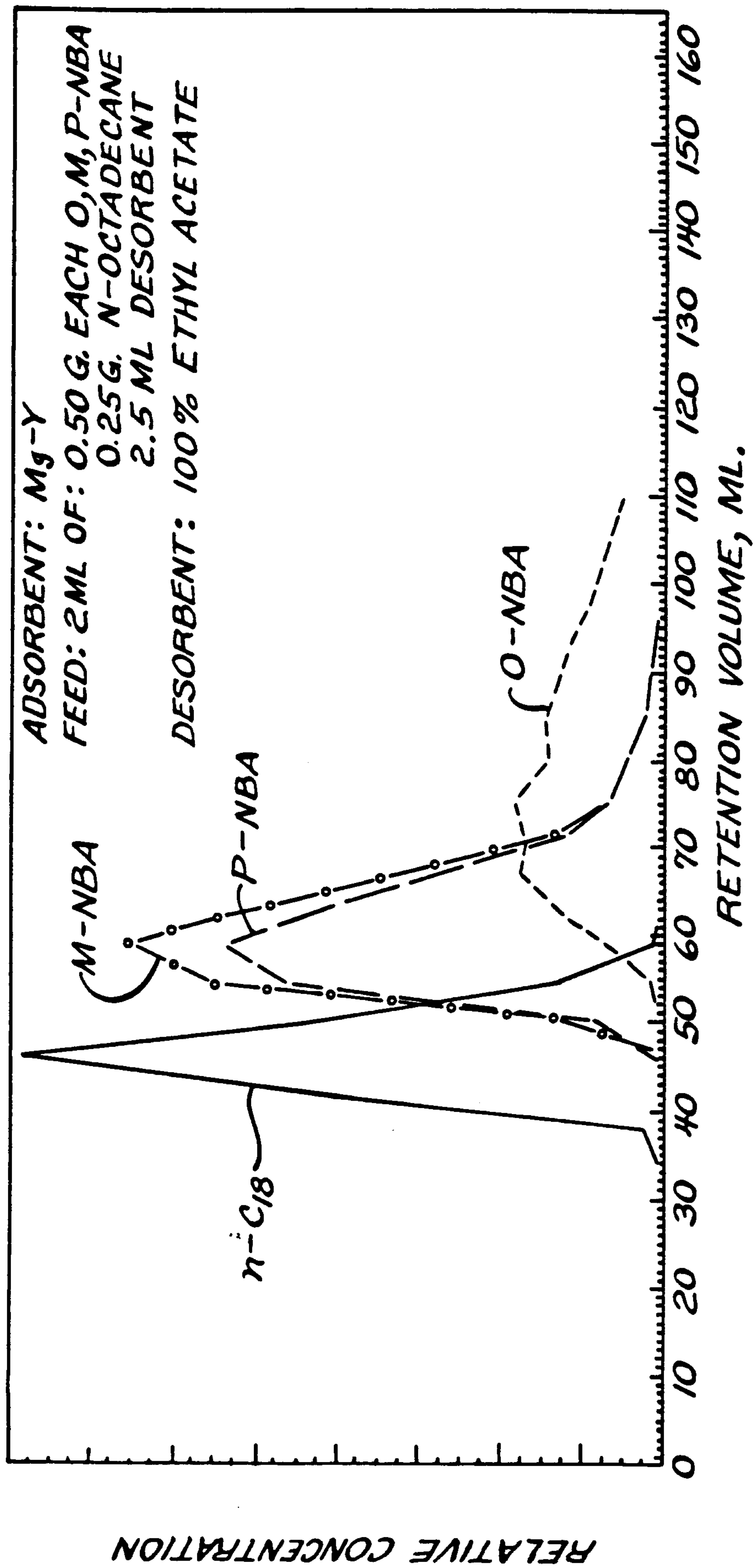
ADSORBENT: Mg-Y
FEED: 2ML OF: 0.50 G. EACH O,M,P-NBA
0.25 G. N-OCTADECANE
2.5 ML DESORBENT
DESORBENT: 100% ETHYL ACETATE
M-NBA
P-NBA
O-NBA
n-C18
RETENTION VOLUME, ML.
RELATIVE CONCENTRATION
0
10
20
30
40
50
60
70
80
90
100
110
120
130
140
150
160

FIG. 9

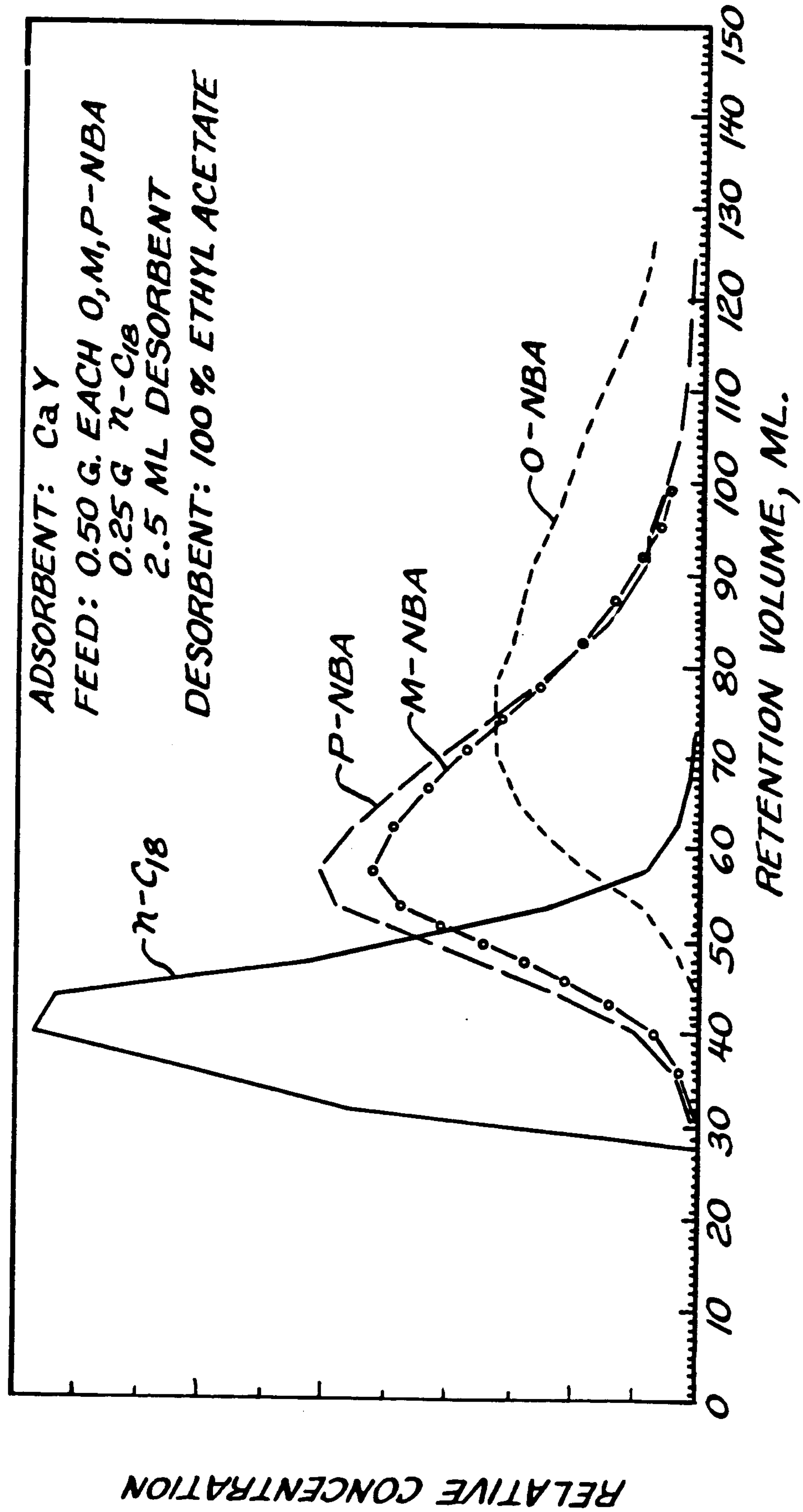
ADSORBENT: CaY
FEED: 0.50 G. EACH O,M,P-NBA
0.25 G n-C18
2.5 ML DESORBENT
DESORBENT: 100% ETHYL ACETATE
n-C18
P-NBA
M-NBA
O-NBA
0
10
20
30
40
50
60
70
80
90
100
110
120
130
140
150
RETENTION VOLUME, ML.
RELATIVE CONCENTRATION

FIG. 10

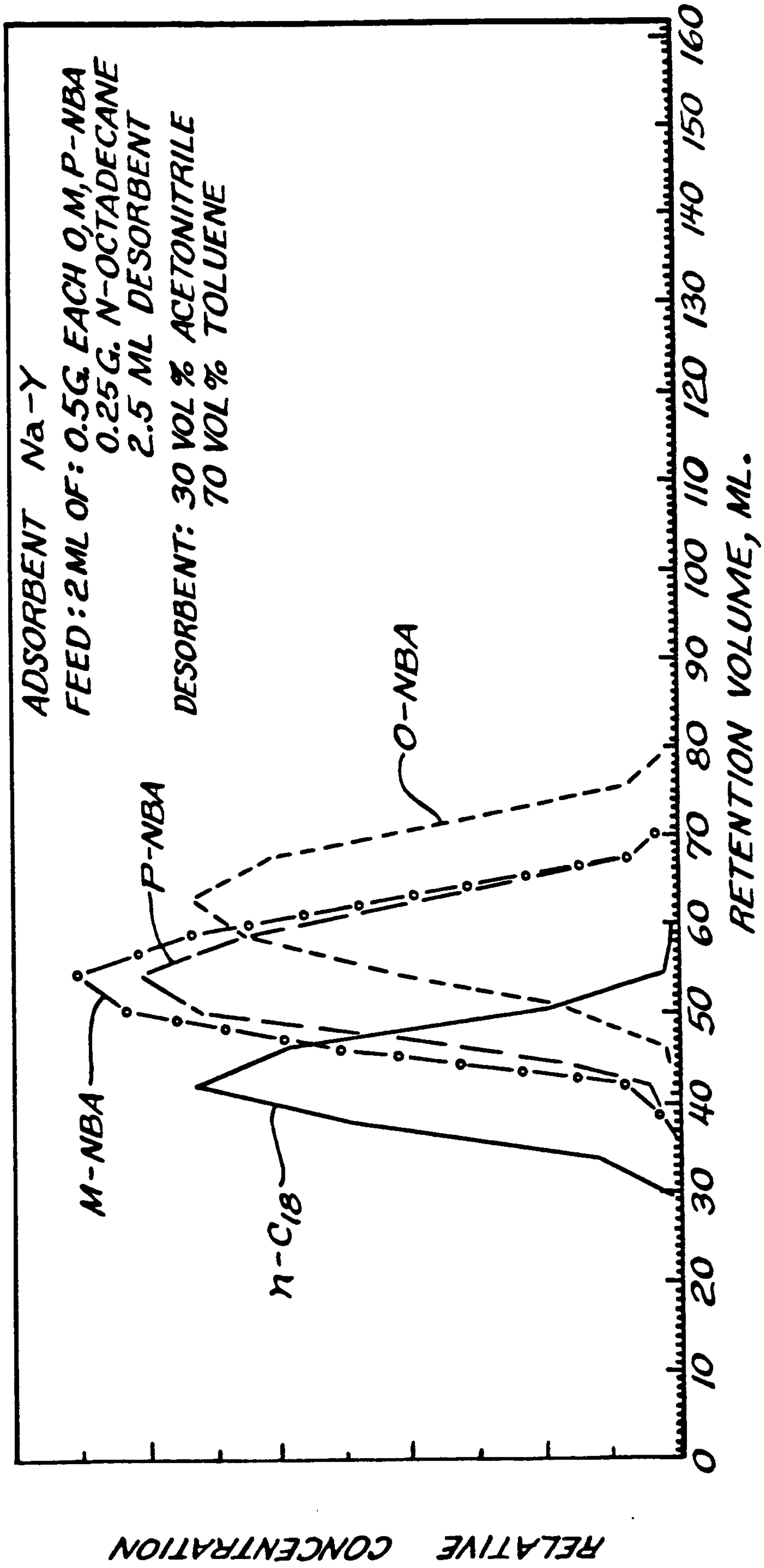
ADSORBENT Na-Y
FEED: 2ML OF: 0.5G. EACH O,M,P-NBA
0.25G. N-OCTADECANE
2.5 ML DESORBENT
DESORBENT: 30 VOL % ACETONITRILE
70 VOL % TOLUENE
O-NBA
P-NBA
M-NBA
n-C18
0
10
20
30
40
50
60
70
80
90
100
110
120
130
140
150
160
RETENTION VOLUME, ML.
RELATIVE CONCENTRATION

FIG. 11

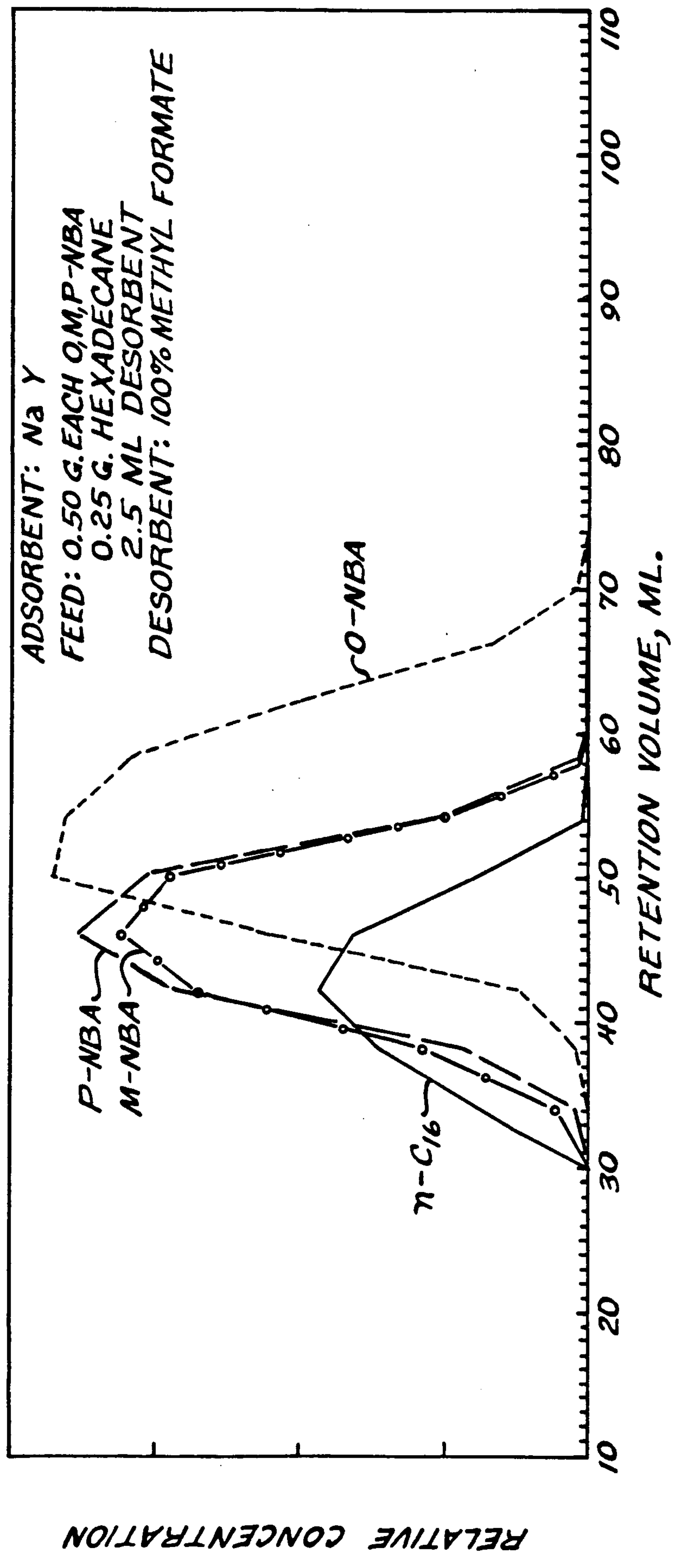
ADSORBENT: Na Y
FEED: 0.50 G. EACH O,M,P-NBA
0.25 G. HEXADECANE
2.5 ML DESORBENT
DESORBENT: 100% METHYL FORMATE
O-NBA
P-NBA
M-NBA
$n$-$C_{16}$
10
20
30
40
50
60
70
80
90
100
110
RETENTION VOLUME, ML.
RELATIVE CONCENTRATION

FIG. 12

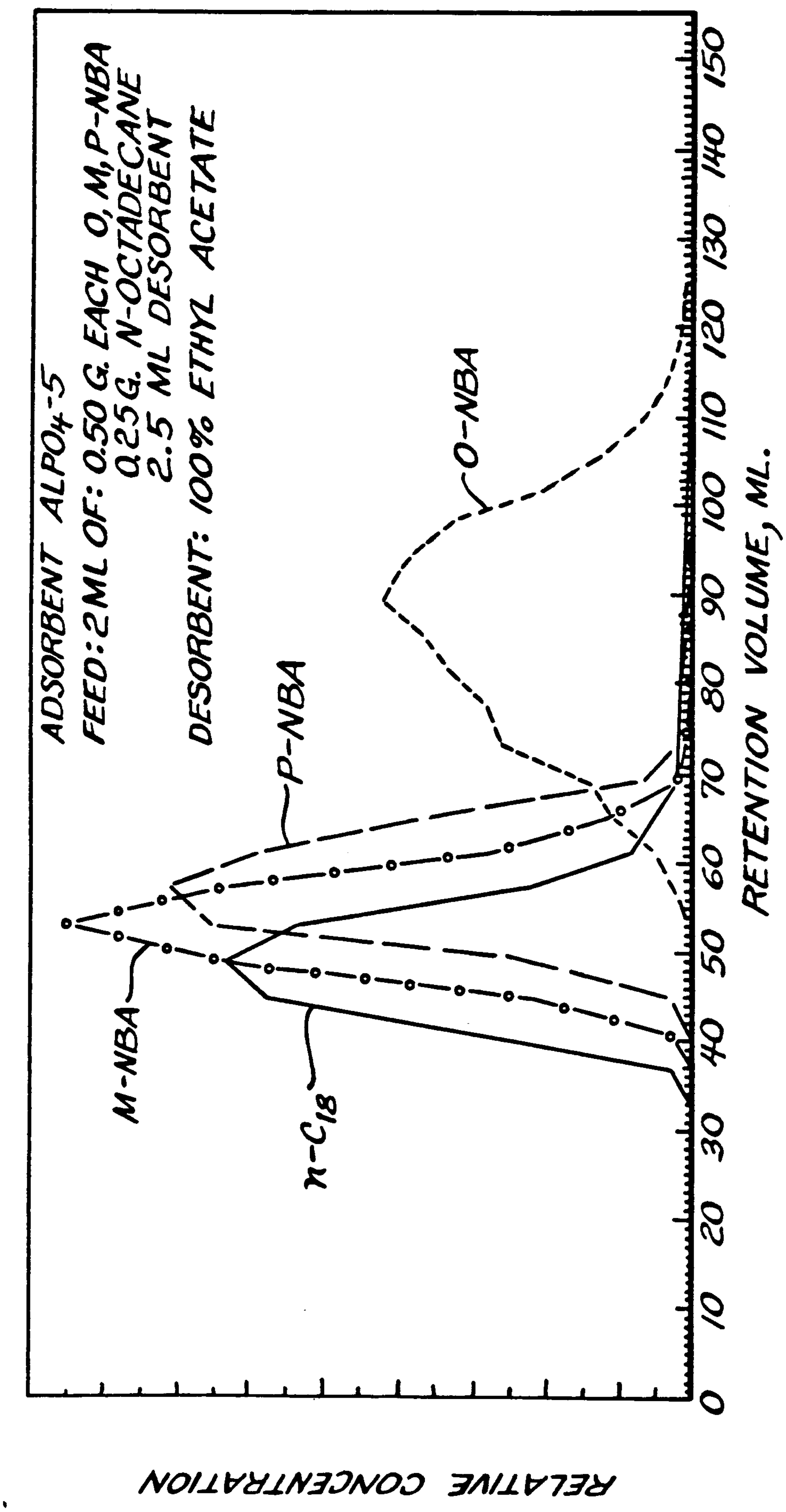
ADSORBENT ALPO4-5
FEED: 2 ML OF: 0.50 G. EACH O, M, P-NBA
0.25 G. N-OCTADECANE
2.5 ML DESORBENT
DESORBENT: 100% ETHYL ACETATE
P-NBA
O-NBA
M-NBA
n-C18
0
10
20
30
40
50
60
70
80
90
100
110
120
130
140
150
RETENTION VOLUME, ML.
RELATIVE CONCENTRATION

FIG. 13